# EUROPEAN PATENT APPLICATION

(11) **EP 1 958 607 A1**
(43) Date of publication of application: **20.08.2008**
(21) Application number: 06781310.5
(22) Date of filing: 20.07.2006
(51) Int. Cl.: A61H 3/00, A61F 2/66, A61F 5/01, A61F 5/02, A63B 23/04

(54) **FORCE TRANSMISSION MEMBER**

(30) Priority: 09.12.2005 JP 2005355505
(71) Applicant: Honda Motor Co., Ltd., Tokyo 107-8556 (JP)
(72) Inventor: SHIMADA, Kei, Wako-shi Saitama 351-0193 (JP)
(74) Representative: Piésold, Alexander James
(86) International application number: PCT/JP2006/314350
(87) International publication number: WO 2007/066427

(57) **Abstract**

Provided is a force transmitting member having a high rigidity without increasing the weight thereof. The force transmitting member (torque transmitting arm 6) for transmitting a force of a power actuator (rotary actuator 4) mounted on an outer side of a lower limb joint to a lower limb part (femoral region T) of a user provides an assist or resistive force to the lower limb. In particular, the force transmitting member comprises a pair of bifurcated portions (bifurcated portions 6c) that are bifurcated from a portion opposing a side of the lower limb obliquely in the forward and rearward directions, respectively, and the bifurcated portions are integrally formed with parts opposing an outer side face, front face and rear face of the lower limb part, respectively. The free end portion of each part opposing the corresponding part of the lower limb part is provided with a joint for a plate (plate 7) that engages the corresponding lower limb part.

## Description

### TECHNICAL FIELD

The present application claims priority from Japanese application No. 2005-355505 filed December 9, 2005, and the contents of this Japanese patent application are incorporated in the present application by reference.

The present invention relates to a force transmitting member, and in particular to a force transmitting member for transmitting force from a power actuator secured to a side of a human joint (such as hip joint and knee joint) to a lower limb part (such as calf and thigh) of a user to provide an assist power or resistive power to the lower limb.

### BACKGROUND OF THE INVENTION

As a device for aiding rehabilitative therapy, it is proposed to mount a power actuator directly on a leg part of a user in WO 95/01141 (Patent Document #1). The structure of the device disclosed in Patent Document #1 comprises a constant-torque drive assembly for applying a load to the flexing movement of a knee, a pair of arms extending upward and downward, respectively, from the drive assembly, a thigh-engaging brace secured to a free end of the upper arm and a calf-engaging brace secured to a free end of the lower arm. By securing the thigh-engaging brace and calf-engaging brace to the thigh and calf of the user, respectively, the constant-torque drive assembly is held in position on a side of the knee joint of the user.

In this device, the arms and braces consist of separate members, and each brace is secured to the corresponding arm via a clamp using a thread mechanism. Each brace is provided with a shape of letter-U having an inwardly directed open end as seen from above, and a support portion fitted with a pad for engaging the thigh or calf is secured to each free end of the brace by using a threaded fastener.

According to the structure proposed in Patent Document #1, to ensure an adequate mechanical strength and rigidity to each arm, the cross sectional area of the arm has to be increased, and this caused the overall weight of the device to be increased to a significant extend if the arms are to be given with an adequate mechanical strength and rigidity.

### BRIEF SUMMARY OF THE INVENTION

### TASKS TO BE ACCOMPLISHED BY THE INVENTION

However, because such a device is typically used by a person having reduced muscle power, it is desirable to minimize the weight of the device and make it easier to handle.

The present invention is based on such a recognition, and, accordingly, has a primary object to provide a force transmitting member that can minimize the weight without reducing the mechanical strength and rigidity.

### MEANS TO ACCOMPLISH THE TASKS

To achieve such an object the present invention provides a force transmitting member for transmitting a force of a power actuator mounted on an outer side of a lower limb joint to a lower limb part of a user via a front support plate and rear support plate that engage a front and rear face of the lower limb part, respectively, for providing an assist or resistive force to the lower limb part, characterized by that: the force transmitting member comprises a pair of bifurcated portions that extend obliquely from an upper part of the force transmitting member to the front and rear support plates, respectively.

As the force transmitting member transmits the force that is applied to the front and rear faces of the lower limb part via the obliquely extending bifurcated portions thereof, a high mechanical strength and rigidity can be achieved by using a minimum amount of material. In particular, it is preferable if each bifurcated portion comprises a free end portion that is curved inward so as to face a front or rear surface of the lower limb part.

If the force transmitting member consists of a one-piece integral member, there are no overlapping portions of different members so that the weight of the force transmitting member can be minimized.

A human lower limb is generally provided with a circular or elliptic cross section. Therefore, if each bifurcated portion is given with a profile defined by a section of an ellipsis, the force transmitting member can favorably conform to the outer profile of the lower limb so that the comfort of the use is enhanced and the mechanical strength of the force transmitting member can be increased in an efficient manner. Also, the outward protrusion of the force transmitting member during use can be minimized.

The force transmitting member may be given with the shape of inverted letter-V, and the upper converging part of the force transmitting member may be connected to the power actuator. However, it is more preferable if the force transmitting member further comprises a main arm portion that extends downwardly from the power actuator and the bifurcated portions are connected to a lower end of the main arm portion so that the force transmitting member is provided with a shape of inverted letter-Y as seen from sideways.

### BRIEF DESCRIPTION OF THE DRAWINGS

Now the present invention is described in the following with reference to the appended drawings, in which:
Figure 1 is a perspective view showing the overall structure of a walking assistance device embodying the present invention;
Figure 2 is a perspective view of the torque transmitting arm given as an example of the force transmitting member of the present invention;
Figure 3 is a side view of the torque transmitting arm;
Figure 4 is a bottom view of the torque transmitting arm; and
Figure 5 is a front view of the torque transmitting arm.

### DETAILED DESCRIPTION OF THE PREFERRED EMBODIMENTS

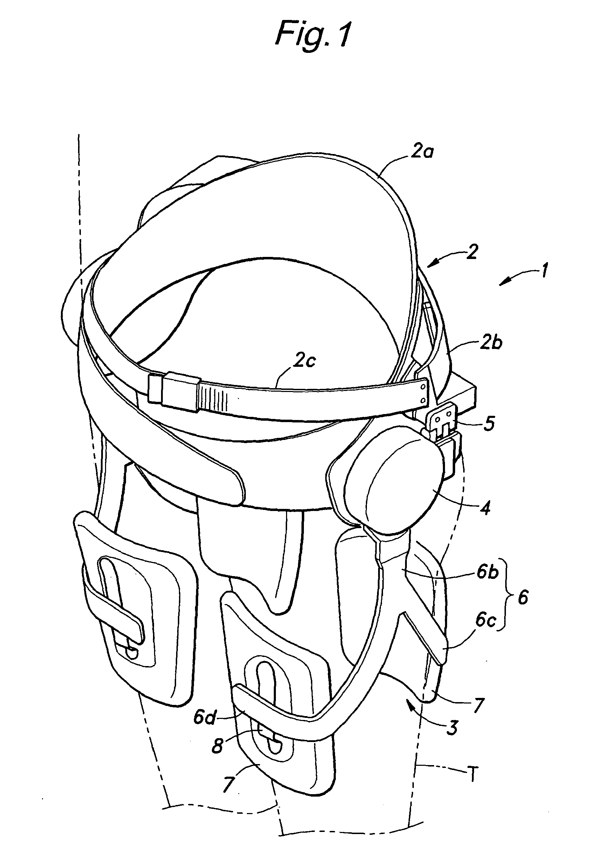

Figure 1 is a simplified view of a walking assistance device embodying the present invention. This walking assistance device 1 comprises a hip support member 2, a pair of femoral support members 3 and a rotary actuator 4 attached to the hip support member 2 for producing an assist power for each of the femoral support members 3. Each rotary actuator 4 is secured to a side of a hip joint of the wearer by securing the hip support member 2 to the hip of the wearer. By wearing the femoral support member 3 on each femoral region T of the wearer, the assist force of the rotary actuator 4 is transmitted to the femoral region T. The device shown in Figure 1 includes a pair of rotary actuators 4 so as to correspond to the two legs of the wearer, but may also include a single rotary actuator that corresponds to only one of the two legs.

The hip support member 2 comprises a hip protective belt 2a made of such material as relatively thick fabric and a back frame 2b made of essentially rigid material so that the hip support member 2 may be worn by the wearer by wrapping the hip protective belt 2a around the hip of the wearer, and with the back frame 2b placed against the backside of the wearer over the hip protective belt 2a, tightening a fastening belt 2c that is connected to the two ends of the back frame 2b.

Each rotary actuator 4 is attached to a corresponding side end of the back frame 2b via a hinge 5 having a hinge pin extending in the fore-and-aft direction so that the laterally outward movement of the femoral region T may be accommodated and the mounting of the femoral support member 3 on the femoral region T may be facilitated.

The femoral support member 3 comprises a torque transmitting arm 6 for transmitting the output torque of the rotary actuator 4 to the femoral region and a pair of support plates 7 each consisting of a thin plate member connected to the torque transmitting arm 6 to interpose the femoral region T from rear and front to apply a suitable pressure to the front and rear faces of the femoral region T. The contact surface of each support plate 7 engaging the femoral region T is fitted with a cushion pad not shown in the drawings.

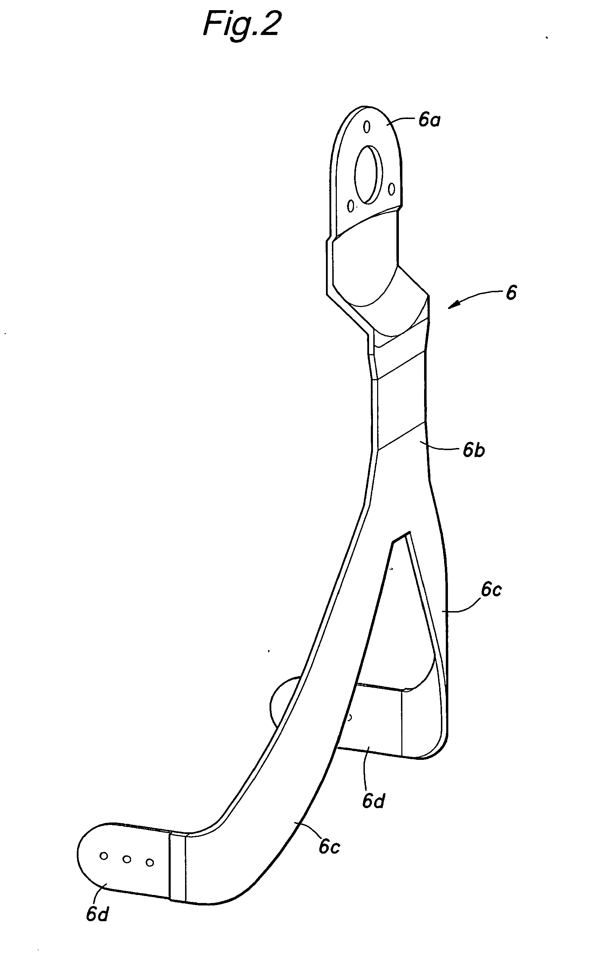

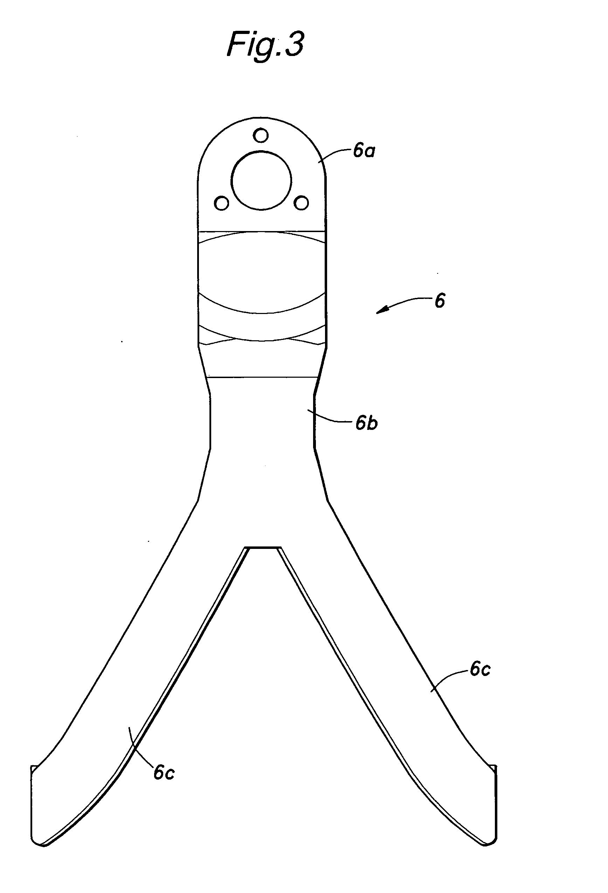

As shown in Figures 2 to 5, the torque transmitting arm 6 comprises a flat base portion 6a connected to a rotatable output end of the rotary actuator 4, a main arm portion 6b depending from the base portion 6a and laterally outwardly offset from the base portion 6a by a distance corresponding to the thickness (axial dimension) of the rotary actuator 4 and a pair of bifurcated portion 6d that are bifurcated from the lower end of the main arm portion 6b obliquely to the front and rear, respectively. Thereby, the torque transmitting arm 6 is generally shaped like inverted letter-Y. The free end of each bifurcated portion 6d is bent inward or toward the lower limb of the user so as to oppose the front or rear face of the femoral portion T, as the case may be, and fixedly carries the corresponding support plate 7 as mentioned earlier.

The outer face of each support plate 7 is provided with a resilient connecting plate 8 consisting of a vertically elongated sheet spring having upper and lower ends connected to the support plate 7. A middle point of the connecting plate 8 which is spaced from the corresponding support plate 7 is connected to the free end portion 6d of the corresponding bifurcated portion 6c via a connecting portion that includes a bolt and nut and a plurality of holes for selectively receiving the bolt in one of the holes so that the point of connection may be varied depending on the build of the wearer.

The back frame 2b, torque transmitting arm 6 and support plates 7 may each consist of a plastic member (FRP) reinforced by carbon or Kevlar so that a high rigidity and a small weight may be accomplished at the same time.

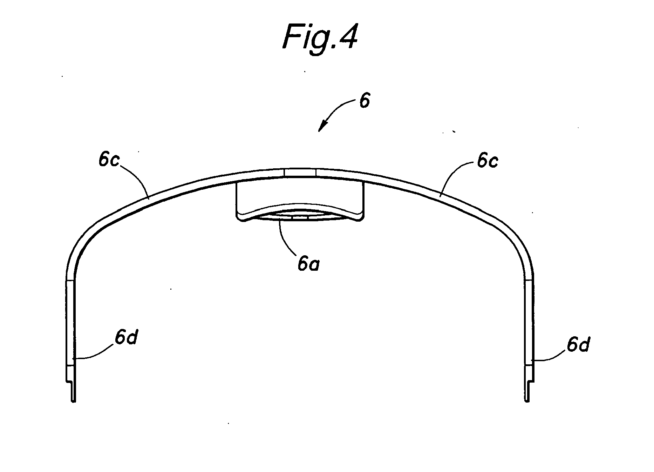

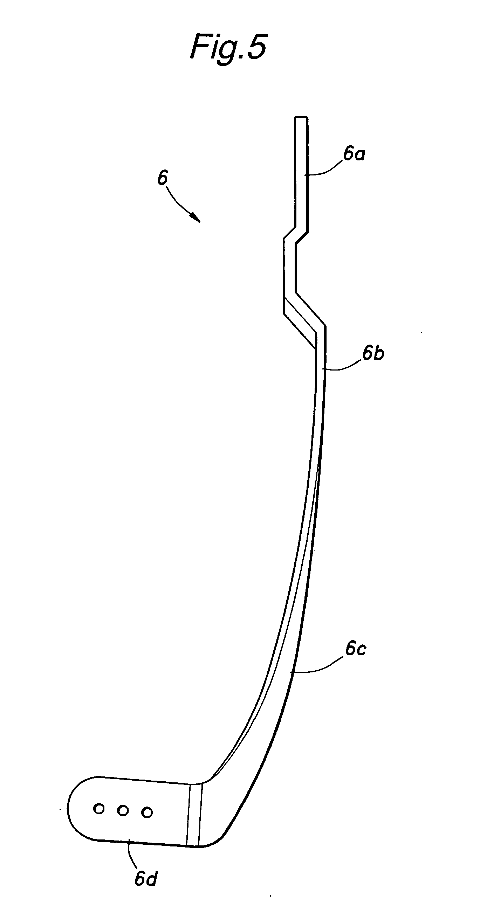

The torque transmitting arm 6 that integrally combines the base portion 6a, main arm portion 6b, bifurcated portions 6c and free end portions 6d is provided with the shape of letter-U with an open side thereof facing inward as seen from above as illustrated in Figure 4. As also shown in Figure 5, the profile of the torque transmitting arm 6, in particular each bifurcated portion 6c of the torque transmitting arm 6 is defined by a section of an ellipsis having a certain curvature so as to conform to the outer profile of the femoral portion.

The bifurcated portions 6c are subjected to bending stress and shear stress as the femoral region T is moved in the fore-and-aft direction. However, because each bifurcated portion 6c extends obliquely downward from the main arm portion 6b, both the horizontal and vertical sections of each bifurcated portion 6c can be given with high moduli of section without increasing the cross sectional area thereof. Therefore, the mechanical strength and rigidity can be increased without excessively increasing the weight.

The torque transmitting arm 6 may consist of a single plate member, but an increased mechanical strength and rigidity may be achieved without increasing the weight by forming it with a layered structure that includes a honeycomb middle layer and a pair of thin plates interposing the middle layer between them. Although not shown in the drawings, ribs and flanges may be formed in various parts of the torque transmitting arm 6 for reinforcement.

The torque transmitting arm 6 was provided with the shape of inverted letter-Y as seen from sideways in the foregoing embodiment, but the present invention is not limited by this embodiment as long as a pair of arms (bifurcated portions 6c) extend obliquely in the forward and rearward directions from a portion (base portion 6a) connected to the rotary actuator 4. For instance, the torque transmitting arm 6 may be given with the shape of inverted letter-V. Also, the illustrated configurations of the hip support member 2 and support plates 7 including the pads are only exemplary, and therefore do not limit the present invention. The rotary actuator was mounted on a side of a hip bone in the illustrated embodiment, but the present invention is equally applicable to a device in which the rotary actuator is mounted on a side of a knee joint. The power actuator that may be used in connection with the present invention is not limited to the illustrated rotary actuator, but may also consist of a spring or a rotary damper that produces a resistive force by using viscous fluid.

### GLOSSAY

| | | | |
|---|---|---|---|
| 1 | walking assistance device | 3 | femoral support member |
| 4 | rotary actuator | 6 | torque transmitting arm |
| 6b | main arm portion | 6c | bifurcated portion |
| 6d | free end portion | T | femoral region |

## Claims

1. A force transmitting member for transmitting a force of a power actuator mounted on an outer side of a lower limb joint to a lower limb part of a user via a front support plate and rear support plate that engage a front and rear face of the lower limb part, respectively, for providing an assist or resistive force to the lower limb part, **characterized by** that:
the force transmitting member comprises a pair of bifurcated portions that extend obliquely from an upper part of the force transmitting member to the front and rear support plates, respectively.

2. The force transmitting member according to claim 1, wherein each bifurcated portion comprises a free end portion that is curved inward so as to face a front or rear surface of the lower limb part.

3. The force transmitting member according to claim 1, wherein the force transmitting member consists of a one-piece integral member.

4. The force transmitting member according to claim 1, wherein each bifurcated portion is given with a profile defined by a section of an ellipsis.

5. The force transmitting member according to claim 1, wherein the lower limb joint is a hip joint, and the lower limb part is a thigh of a user.

6. The force transmitting member according to claim 1, wherein the force transmitting member further comprises a main arm portion that extends downwardly from the power actuator and the bifurcated portions are connected to a lower end of the main arm portion so that the force transmitting member is provided with a shape of inverted letter-Y as seen from sideways.
